(19) Europäisches Patentamt
European Patent Office
Office européen des brevets

(11) Veröffentlichungsnummer: **0 142 094**
**B1**

# (12) EUROPÄISCHE PATENTSCHRIFT

(45) Veröffentlichungstag der Patentschrift: **29.06.88**

(51) Int. Cl.⁴: **C 07 H 13/00**

(21) Anmeldenummer: **84113006.5**

(22) Anmeldetag: **29.10.84**

**(54) Verfahren zur Herstellung von acylierten Zuckern mit glycosidisch gebundener Isthiocyanatgruppe.**

(30) Priorität: **12.11.83 DE 3341018**

(43) Veröffentlichungstag der Anmeldung: **22.05.85 Patentblatt 85/21**

(45) Bekanntmachung des Hinweises auf die Patenterteilung: **29.06.88 Patentblatt 88/26**

(84) Benannte Vertragsstaaten: **BE CH DE FR GB IT LI**

(56) Entgegenhaltungen:
**CHEMICAL ABSTRACTS, Band 96, Nr. 17, 26. April 1982, Seite 796, Nr. 143202k, Columbus, Ohio, US; OGURA, HARUO et al.: "Studies on nucleoside analogs. XXIV. Mono- and disaccharide isothiocyanates"**
**SYNTHESIS, Nr. 6, Juni 1984, Seiten 509-510; J. CAMARASA et al.: "A new procedure for the synthesis of glycosyl isothiocyanates"**

(73) Patentinhaber: **BAYER AG, Konzernverwaltung RP Patentabteilung, D-5090 Leverkusen 1 Bayerwerk (DE)**

(72) Erfinder: **Hassel, Tillmann, Dr., Morgengraben 14, D-5000 Köln 80 (DE)**
Erfinder: **Müller, Hanns Peter, Dr., Im Kerberich 6, D-5068 Odenthal (DE)**

**EP 0 142 094 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft ein Verfahren zur Herstellung von acylierten Zuckern mit glycosidisch gebundener Isothiocyanatgruppe unter Verwendung von Phasentransferkatalysatoren.

Acylierte Zucker mit glycosidisch gebundener Isothiocyanatgruppe sind an sich bekannt. Man stellt sie her, indem man acylierte Zucker mit glycosidisch gebundenem Halogenatom mit bestimmten Schwermetallrhodaniden umsetzt. So setzt man nach der Japan Kokai No. 77,195,123, als Schwermetallrhodanid Silberrhodanid ein. In Heterocycles 17, 87 (1982) wird die Verwendung von Bleirhodanid für den gleichen Zweck beschrieben. Bei der Verwendung von Ammoniumrhodanid entsteht nicht wie früher angenommen (CA 91,14117d (1978)) das gewünschte Isothiocyanat, sondern das entsprechende Thiocyanat (Heterocycles 17, 87 (1982)).

Bei den bekannten Verfahren zur Herstellung von acylierten Zuckern mit glycosidisch gebundener Isothiocyanatgruppe werden nachteiligerweise Schwermetallrhodanide eingesetzt. Diese schon an sich teuren Schwermetallrhodanide sind keine technisch verfügbaren Substanzen, sondern erfordern zu ihrer Herstellung aufwendige Herstellungsverfahren. Hinzu kommt, daß Schwermetallverbindungen im allgemeinen toxisch sind und besondere Aufbereitungsverfahren erfordern.

Es wurde ein Verfahren zur Herstellung von acylierten Zuckern mit glycosidisch gebundener Isothiocyanatgruppe aus acylierten Zuckern mit glycosidisch gebundenem Halogenatom gefunden, bei dem man die acylierten Zucker mit glycosidisch gebundenem Halogenatom mit wenigstens der äquivalenten Menge eines Rhodanids der Formel

Me - NCS (I),

in der

Me Alkali, Erdalkali oder Ammonium bedeutet,

in einem inerten Lösungsmittel bei erhöhter Temperatur in Gegenwart eines Phasentransferkatalysators umsetzt.

Nach dem erfindungsgemäßen Verfahren erhält man acylierte Zucker mit glycosidisch gebundener Isothiocyanatgruppe ohne Einsatz von Schwermetallrhodaniden. Nach dem erfindungsgemäßen Verfahren erhält man überraschenderweise nicht, wie man aus Maygar Biol. Kutató Munkái 13, 525 (1941) und Synthesis 184 (1977) erwarten konnte, die Thiocyanate sondern die Isothiocyanate. Erfindungsgemäß erhält man die acylierten Zucker mit glycosidisch gebundener Isothiocyanatgruppe mit hohen Ausbeuten und großer Reinheit.

Das erfindungsgemäße Verfahren kann anhand der folgenden Reaktionsgleichung erläutert werden:

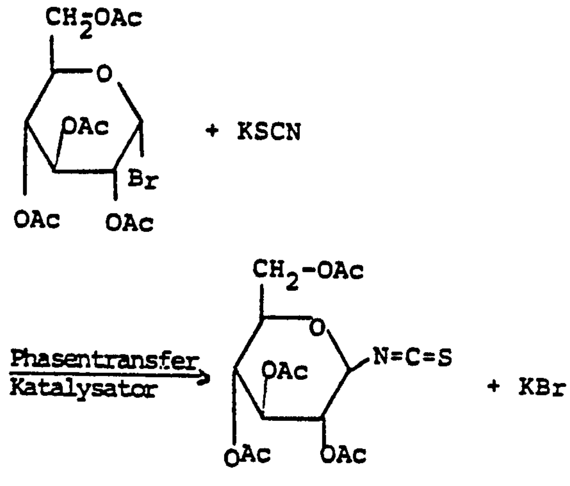


wobei Ac für $-\overset{O}{\overset{\|}{C}}-CH_3$ steht.

Acylierte Zucker mit glycosidisch gebundenem Halogenatom sind an sich bekannt (F. Micheel, Chemie der Zucker und Polysaccaride, 2. Auflage 1956). S. 455-456, S. 476-477; Geest und Portig, Leipzig 1956). Sie können beispielsweise hergestellt werden, indem man einen Zucker mit überschüssigem Säureanhydrid erschöpfend acyliert und danach durch Umestern mit wasserfreiem Halogenwasserstoff die Estergruppe am glycosidischen Kohlenstoffatom durch das Halogenatom des eingesetzten Halogenwasserstoffs ersetzt.

Das glycosidisch gebundene Halogenatom kann Fluor, Chlor, Brom oder Jod, bevorzugt Chlor oder Brom, sein.

Acylierte Zucker mit glycosidisch gebundenem Halogenatom sind beispielsweise Verbindungen der Formel

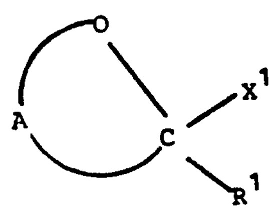
(II),

in der

$R^1$ Wasserstoff oder Acyloxymethylen bedeutet,

A für einen der Reste

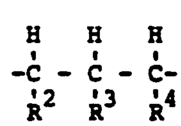 oder 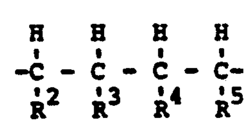 steht,

in denen

$R^2$ bis $R^5$ gleich oder verschieden sind und Wasserstoff, Acylamino, Acylthio, Nitro, Azido, Organosulfonyloxy, Acylester und

Acyloxymethylen bedeuten und wobei einer der Reste $R^2$ bis $R^5$ auch ein weiterer Zuckerrest der Formel

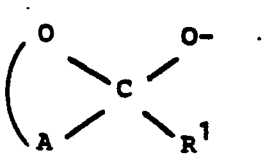

sein kann,
in dem
$R^1$ und A die obengenannte Bedeutung haben, und
$X^1$ Halogen bedeutet.

In den Resten Acyl $(R-\overset{O}{\overset{\|}{C}}-)$, Acylthio $(R-\overset{O}{\overset{\|}{C}}-S-)$, Organosulfonyloxy $(R-\underset{O}{\overset{O}{\overset{\|}{\underset{\|}{S}}}}-O)$, Acylamino $(R-\overset{O}{\overset{\|}{C}}-NH-)$, Acylester $(R-\overset{O}{\overset{\|}{C}}-O-)$ und Acyloxymethylen $(R-\overset{O}{\overset{\|}{C}}-OCH_2-)$ bedeutet hierbei der organische Rest (R) im allgemeinen einen geradkettigen oder verzweigten Kohlenwasserstoffrest mit 1 bis 12, bevorzugt 1 bis 8, Kohlenstoffatomen. Bevorzugte Reste sind Niederacyl, Niederacylthio, Niederorganosulfonyloxy, Niederacylamino, Niederacylester und Niederacyloxymethylen mit 1 bis etwa 6 Kohlenstoffatomen im organischen Rest.

Beispielsweise seien die folgenden Reste genannt: Acylreste, wie Acetyl, Propionyl, Pivaloyl, Benzoyl, 4-Methylbenzoyl.

Acylthioreste, wie Acetylthio, Butyrylthio, Benzoylthio.

Organosulfonyloxy, wie Methansulfonyloxy, Benzolsulfonyloxy, 4-Methylbenzolsulfonyloxy.

Acylamino, wie Acetylamino, 2-Methylpropionylamino, Benzoylamino, Butyrylamino.

Acylester, wie Acetoxy, Propionyloxy, Benzoyloxy, Pivaloyloxy, Butyryloxy und

Acyloxymethylen, wie Acetoxymethylen, Benzoyloxymethylen, Propionyloxymethylen, Butyryloxymethylen, Pivaloyloxymethylen.

Es ist auch möglich, daß der Zuckerrest mit weiteren Zuckerresten zu Disacchariden, Oligosacchariden oder Polysacchariden verknüpft ist. Bevorzugt für das erfindungsgemäße Verfahren sind jedoch Mono-, Di- und Oligosaccharide mit 1 bis 6, bevorzugt 1 oder 2, Zuckerresten im Molekül.

Bevorzugte acylierte Zucker mit glycosidisch gebundenem Halogenatom für das erfindungsgemäße Verfahren sind Verbindungen der Formel

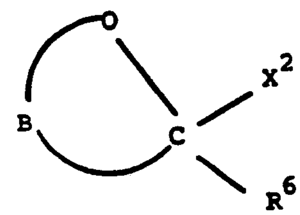

(III),

in der

$R^6$ Wasserstoff oder Niederacyloxymethylen bedeutet,

B für einen der Reste

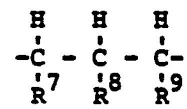

oder

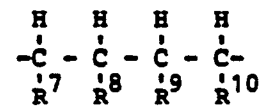

steht,

in denen

$R^7$ bis $R^{10}$ gleich oder verschieden sind und Wasserstoff, Niederacylamino, Niederacylester, Niederorganosulfonyloxy und Niederacyloxymethylen bedeuten und wobei einer der Reste $R^7$ bis $R^{10}$ auch ein weiterer Zuckerrest der Formel

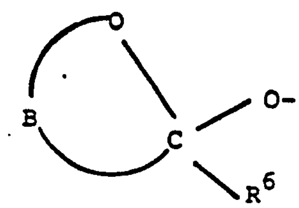

sein kann,
in dem
$R^6$ und B die oben genannte Bedeutung haben,

und
$X^2$ Chlor oder Brom bedeutet.

Im einzelnen seien die folgenden acylierten Zucker mit glycosidisch gebundenem Halogenatom genannt: 2, 3, 4, 6-Tetraacetyl-D-glucopyranosylbromid; 2, 3, 4-Tribenzoyl-D-ribopyranosylbromid; 2, 3, 5-Tribenzoyl-D-ribofuranosylbromid; 2, 2', 3, 3', 4', 6, 6'-Heptaacetyl-D-lactosylbromid; 2, 2', 3, 3', 4', 6, 6'-Heptaacetyl-D-cellobiosylbromid; 2, 2', 3, 3', 4', 6, 6'-Heptaacetyl-D-maltosyl bromid, aber auch 2-Acetamido-2-desoxy-3, 4, 6-triacetyl-D-glucopyranosylchlorid und 2-(4-Methyl)benzolsulfonyl-oxy-2-desoxy-3, 4, 6-triacetyl-D-glucopyranosylbromid.

Rhodanide für das erfindungsgemäße Verfahren sind Alkali-, Erdalkali-oder Ammoniumrhodanide. Alkali und Erdalkali im Rahmen des erfindungsgemäßen Verfahrens sind

die Metalle der ersten und zweiten Gruppe des Periodensystems nach Mendelejew. Bevorzugt seien Lithium, Natrium und Kalium genannt.

Bevorzugte Rhodanide für das erfindungsgemäße Verfahren sind Ammoniumrhodanid, Natriumrhodanid und Kaliumrhodanid.

Die Rhodanide werden in das erfindungsgemäße Verfahren in äquivalenter Menge oder im Überschuß eingesetzt. Im allgemeinen setzt man 1 bis 10, bevorzugt 1 bis 5, Äqivalente des Rhodanids bezogen auf den acylierten Zucker mit glycosidisch gebundenem Halogenatom ein.

Das erfindungsgemäße Verfahren wird in Gegenwart von inerten Lösungsmitteln, also Lösungsmitteln, die sich unter den Reaktionsbedingungen nicht verändern, durchgeführt. Bevorzugt kann das erfindungsgemäße Verfahren in inerten Lösungsmitteln durchgeführt werden, in denen sich das Rhodanid praktisch nicht löst. Unter "praktisch nicht lösen" wird erfindungsgemäß eine Löslichkeit unter 0,1 % verstanden.

Lösungsmittel für das erfindungsgemäße Verfahren sind beispielsweise

geradkettige, verzweigte oder cyclische aliphatische Ether wie Dibutylether und Dioxan,

aliphatische Nitrile wie Acetonitril,

Ester und Ketone wie Butylacetat und Propanon,

aromatische Kohlenwasserstoffe wie Benzol und

araliphatische Kohlenwasserstoffe wie Toluol, Ethylbenzol und die isomeren Xylole.

Bevorzugte Lösungsmittel für das erfindungsgemäße Verfahren sind aromatische und araliphatische Kohlenwasserstoffe. Insbesondere seien Benzol, Toluol, Ethylbenzol und Xylol genannt.

Selbstverständlich können die Lösungsmittel auch als Gemische eingesetzt werden.

Im allgemeinen verwendet man 1 bis 20, bevorzugt 2 bis 12, Gewichtsteile des inerten Lösungsmittels bezogen auf das eingesetzte Zuckerderivat.

Das erfindungsgemäße Verfahren wird in Gegenwart eines Phasentransferkatalysators durchgeführt, der bevorzugt eine Komplexierungskonstante von 170 L/mol bis $10^8$ L/mol hat. Insbesondere werden Phasentransferkatalysatoren mit einer Komplexierungskonstante von 450 L/mol bis $10^6$ L/mol bevorzugt.

Als Komplexierungskonstante ist hier die Gleichgewichtskonstante für die Bildung von Ionenpaaren mit eingeschobenem Phasentransferkatalysator bei Lösungen von Fluorenylnatrium in Tetrahydrofuran oder Tetrahydropyran zu verstehen (Kontakte 1977, Heft 2, Seite 20 ff).

Bevorzugte Phasentransferkatalysatoren für das erfindungsgemäße Verfahren sind Polyether.

Die Polyether für das erfindungsgemäße Verfahren sind aus Ethylenoxideinheiten aufgebaut und können linear oder cyclisch sein.

Lineare Polyether als erfindungsgemäße Phasentransferkatalysatoren werden im allgemeinen mit einem Molekulargewicht von 150 bis 2000, bevorzugt von 190 bis 1000, eingesetzt.

Beispielsweise seien die folgenden linearen Polyether genannt:

Tetraethylenglykol, Hexaethylenglykol, Octaethylenglykol, Decaethylenglykol.

Die linearen Polyether können auch in Form technischer Gemische mit mittleren Molgewichten von 200 bis 2000, bevorzugt von 400 bis 1000, eingesetzt werden.

Beispielsweise seien die folgenden Polyether genannt:

Polyethylenglykol $\bar{M}$ = 400
Polyethylenglykol $\bar{M}$ = 650,
Polyethylenglykol $\bar{M}$ = 1000.

Die H-Atome der endständigen OH-Gruppen der Polyether können gegebenenfalls durch Alkylreste ersetzt sein. Die Alkylreste können linear oder verzweigt mit einer Kohlenstoffzahl von 1 bis 6 sein. Bevorzugt sind lineare Alkylreste mit einer Kohlenstoffzahl von 1 und 2. Beispielsweise seien die folgenden Polyether mit endständigen Alkylgruppen genannt:

Hexaethylenglykoldimethylether
Polyethylenglykol $\bar{M}$ = 400-dimethylether,
Polyethylenglykol $\bar{M}$ = 650-diethylether,
Octaethylenglykoldipropylether.

Polyether als erfindungsgemäße Phasentransferkatalysatoren können auch makrocyclische Polyether mit einer Ringgröße von 12 bis 24 Atomen, bevorzugt von 15 bis 18 Atomen, sein.

In diesen makrocyclischen Polyethern können auch eine oder mehrere, bevorzugt eine und zwei, Ethylengruppen durch ein oder mehrere, bevorzugt ein oder zwei aromatische und/oder aliphatische Ringsysteme der Kohlenstoffzahl 5 bis 10, bevorzugt 6, derart ersetzt sein, daß die an die jeweils ersetzten Ethylengruppen gebundenen Sauerstoffatome durch zwei Kohlenstoffatome des entsprechenden aromatischen oder aliphatischen Ringsystems getrennt sind. Derartige Polyether sind beispielsweise in der US-PS-3 686 225 beschrieben.

Beispielsweise seien die folgenden makrocyclischen Polyether genannt:

1.4.7.10-Tetraoxacyclododecan, 1.4.7.10.13-Pentaoxacyclopentadecan, 1.4.7.10.13.16-Hexaoxacyclooctadecan, 2.5.8.15.18.21-Hexaoxatricyclo[20.4.0.0$^{9.14}$] hexacosan, 2.5.8.11.14.17-Hexaoxabicyclo[16.4.0.] docosan, 2.3.11.12-Dibenzo-1.4.7.10.13.16-hexaoxacyclooctadeca-2.11.-dien, 2.3-Benzo-1.4.7.10.13-pentaoxacyclopentadeca-2-en.

Zur Erleichterung der Wiedergewinnung der erfindungsgemäßen Polyether kann es vorteilhaft sein, diese durch bekannte chemische Reaktionen (beispielsweise Formylierung bei Polyethern mit aromatischen Ringsystem) zu modifizieren, die modifizierten Polyether in ebenfalls an sich bekannter Weise an einen polymeren Träger zu binden und die derart

modifizierten Polyether in das erfindungsgemäße Verfahren einzusetzen.

Insbesondere werden für das erfindungsgemäße Verfahren Phasentransferkatalysatoren wie Polyethylenglykol $\bar{M}$ = 400-dimethylether, Octaethylenglykoldipropylether, Polyethylenglykol $\bar{M}$ = 650-diethylether, 1.4.7.10.13-Penta-oxacyclopentadecan, 1.4.7.10.13.16.-Hexaoxacycloocta decan, 2.5.8.15.18.21-Hexaoxatricyclo[20.10.13.16-Hexaoxacyclooctadecan, 2.5.8.15.18.21.-Hexaoxatricyclo[20.4. $0.0^{9.14}$]hexacosan, 2.3.11.12.-Dibenzo-1.4.7.10.13.16-hexaoxacyclooctadeca-2.11.-dien, 2.3-Benzo-1.4.7.10.13-pentaoxacyclopentadeca-2-en bevorzugt.

Für das erfindungsgemäße Verfahren setzt man im allgemeinen 0,1 bis 10 Mol-%, bevorzugt 1 bis 5 Mol-% des Phasentransferkatalysators bezogen auf den eingesetzten Acylhalogenzucker ein.

Das erfindungsgemäße Verfahren wird bei erhöhter Temperatur durchgeführt. Im allgemeinen führt man das erfindungsgemäße Verfahren im Temperaturbereich von 20 bis 150° C, bevorzugt im Temperaturbereich von 80 bis 110° C, durch.

Das erfindungsgemäße Verfahren wird im allgemeinen bei Normaldruck durchgeführt. Es ist aber selbstverständlich auch möglich, das erfindungsgemäße Verfahren bei einem Unter- oder Überdruck (beispielsweise im Druckbereich von 0,5 bis 10 bar) durchzuführen.

Das Ende der Umsetzung kann mit physikalischen Methoden, beispielsweise spektroskopisch, leicht verfolgt werden. Im allgemeinen ist die Umsetzung nach einer Reaktionszeit von 3 bis 30 Stunden, bevorzugt von 6 bis 24 Stunden, beendet.

Das erfindungsgemäße Verfahren kann beispielsweise wie folgt durchgeführt werden:

Der acylierte Zucker mit glycosidisch gebundenem Halogenatom wird zusammen mit dem Rhodanid und dem Phasentransferkatalysator in dem Lösungsmittel vorgelegt. Man erhitzt das Reaktionsgemisch unter starkem Rühren auf die gewünschte Reaktionstemperatur. Das Reaktionsgemisch wird bis zur Beendigung der Umsetzung gerührt.

Die gewünschten acylierten Zucker mit einer glycosidisch gebundenen Isothiocyanatgruppe werden von der organischen Lösung in an sich bekannter Weise durch Destillation und Rekristallisation isoliert.

Nach dem erfindungsgemäßen Verfahren können acylierte Zucker mit glycosidisch gebundener Isothiocyanatgruppe der Formel

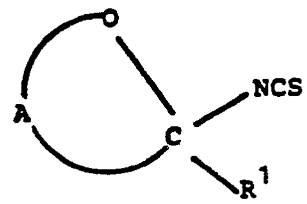 (IV),

in der

$R^1$ Wasserstoff oder Acyloxymethylen bedeutet,

A für einen der Reste

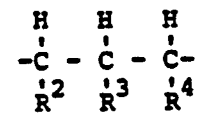 oder

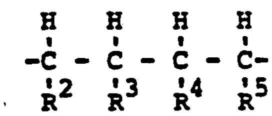 steht,

in denen

$R^2$ bis $R^5$ gleich oder verschieden sind und Wasserstoff, Acylamino, Acylthio, Nitro, Azido, Organosulfonyloxy, Acylester und Acyloxymethylen bedeuten und wobei einer der Reste $R^2$ bis $R^5$ auch ein weiterer Zuckerrest der Formel

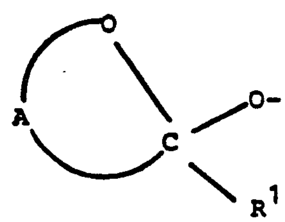

sein kann,

in dem

$R^1$ und A die oben genannte Bedeutung haben,

hergestellt werden.

Acylierte Zucker mit glycosidisch gebundener Isothiocyanatgruppe sind wichtige organischchemische Zwischenprodukte. Sie besitzen entweder bereits selbst Wirkstoffcharakter oder dienen als Komponenten zum Aufbau neuer Wirkstoffe oder zur Modifizierung bekannter Substanzen (DE-OS-2 509 260, EP 24 202 und DD-PS 34 860).

So kann beispielsweise aus dem nach dem erfindungsgemäßen Verfahren erhältlichen 2,3,4,6-Tetracetyl-β-D-glucosyl-1-isothiocyanat in an sich bekannter Weise (Ogura et. al.: Chem. Pharm. Bull. 29, 1938 (1981)) durch Umsetzung mit 5-Methyl-isothioharnstoffiodid das 1-(2,3,4,6-

Tetraacetyl-β-D-glucosyl)-4-5-methyliso-thiobiuret hergestellt werden. Durch Abspaltung der Acetylgruppen nach bekannten Methoden erhält man aus diesem Isothiobiuret das nematizid wirksame 1-β-D-Gluco syl-4-5-methylisothiobiuret (Beispiel 9).

**Beispiel 1**

In einem Kolben mit Magnetrührer und Rückflußkühler mit Trockenrohr legt man 50 g (121,7 mmol) Acetobromglucose, 11,8 g (121,7 mmol) Kaliumthiocyanat und 0,3 g (1,14 mmol) 1.4.7.10.13.16-Hexaoxacyclooctadecan in 150 ml Toluol vor. Man rührt 7 Stunden unter Rückfluß (100° C) und saugt heiß vom Ungelösten ab. Aus dem Filtrat kristallisieren beim Erkalten 32,32 g (83,1 mmol) 2,3,4,6-Tetraacetyl-ß-D-glucosyl-1-isothiocyanat.

Aus der Mutterlauge lassen sich nach Einengen und Umkristallisieren mit Diisopropylether weitere 4,3 g (11,05 mmol) Produkt gewinnen.

Gesamtausbeute 36,62 g (94,14 mmol) = 77,4 % Fp.: 113° C.

**Beispiel 2**

In einem Kolben mit Magnetrührer und Rückflußkühler mit Trockenrohr werden 10 g (24,5 mmol) Acetobromglucose, 2,401 g (24,5 mmol) Kaliumthiocyanat und 100 mg (0,25 mmol) Polyethylenglykol $\bar{M}$ = 400-dimethylether in 25 ml Toluol vorgelegt. Man kocht 20 h unter Rückfluß und saugt heiß ab. Aus dem Filtrat kristallisieren beim Erkalten 6,2 g (15,94 mmol) des Isothiocyanats von Beispiel 1.

Ausbeute 65 %, Fp.: 130° C.

**Beispiel 3 (Vergleichsbeispiel)**

Der Ansatz von Beispiel 2 wird ohne Zusatz eines Phasentransferkatalysators wiederholt. Nach dem Absaugen erhält man aus dem Filtrat keine Fällung. Das aus dem Filtrat durch Abdestillieren des Toluols isolierte Produkt schmilzt unscharf zwischen 86 und 94° C.

**Beispiel 4**

In 180 ml Toluol werden 16 g (22,89 mmol) Acetobromcellobiose, 2,22 g (22,89 mmol) Kaliumrhodanid und 56,5 mg 1.4.7.10.13.16-Hexaoxacyclooctadecan vorgelegt. Man erhitzt 7 Stunden auf 110° C und saugt heiß ab. Aus dem Filtrat kristallisieren beim Erkalten 10,9 g Heptaacetyl-cellobiosyl-1- isothiocyanat vom Fp. 209° C aus.

Ausbeute: 10,9 g ≙ 16,1 mmol ≙ 70,33 %.

**Beispiel 5**

In 70 ml Acetonitril werden 20 g (48,68 mmol) Acetobromglucose, 4,72 g (48,7 mmol) Kaliumrhodanid und 60 mg 1.4.7.10.13.16-Hexaoxacyclooctadecan vorgelegt. Man kocht 8 Stunden unter Rückfluß, nutscht heiß ab und destilliert das Acetonitril aus dem Filtrat im Vakuum ab. Der Rückstand wird aus 10 ml Toluol umkristallisiert und 2 mal mit 5 ml eiskaltem Toluol gewaschen. Man erhält 11 g Produkt.

Ausbeute 11 g ≙ 28,28 mmol ≙ 58 %.

**Beispiel 6**

In 30 ml Toluol werden 10,25 g (25 mmol) Acetobromglucose, 2,1 g (25 mmol) Natriumrhodanid und 55 mg (0,25 mmol) 1.4.7.10.13.16-Hexaoxacyclootadecan vorgelegt. Man erhitzt 7 Stunden auf 110° C und saugt heiß ab. Aus dem Filtrat kristallisieren bei Erkalten 6,76 g des Produktes von Beispiel 1.

Ausbeute: 6,76 g ≙ 17,4 mmol ≙ 69,6 %.

**Beispiel 7**

Der Ansatz von Beispiel 6 wird wiederholt, jedoch wird das Natriumrhodanid durch 1,9 g (25 mmol) Ammoniumrhodanid ersetzt.

Man erhält 4,56 g Produkt von Beispiel 1

Ausbeute: 4,56 g ≙ 11,8 mmol ≙ 47,2 %.

**Beispiel 8**

In 40 ml Toluol werden 4,51 g (8,37 mmol) 2-(4-Methyl)-benzolsulfonyloxy-2-desoxy-3,4,6-triacetyl-D-glucopyranosylbromid, 0,81 g (8,37 mmol) Kaliumrhodanid und 185 mg (0,837 mmol) 1.4.7.10.13.16-Hexaoxyacylooctadecan vorgelegt. Man erhitzt 7 Stunden auf 110° C und saugt ab. Das Filtrat wird auf -20° C gekühlt. Es kristallisieren 1,8 g (3,6 mmol) 2-(4-Methyl)benzolsulfonyl-2-desoxy-3,4,6-triacetyl-β-D-glucopyranosyl-1-iso-thiocyanat.

Ausbeute: 1,8 g ≙ 3,6 mmol ≙ 43 %

Fp.: 150° C

**Beispiel 9** (Verwendungsbeispiel)

1-β-D-Glucosyl-4-5-methylisothiobiuret

Zu einer 40° C warmen Lösung von 20,76 g (95,2mmol) 5-Methyl-isothioharnstoffiodid und 37,03 g (95,2 mmol) 2,3,4,6-Tetra-acetyl-β-D-glucosyl-1-isothiocyanat in 100 ml Acetonitril tropft man 40 ml Triethylamin. Es tritt eine exotherme Reaktion auf. Es wird zur Trockne

eingeengt, der Rückstand wird in Chloroform gelöst und zweimal mit Wasser extrahiert. Die organische Phase wird abgetrennt, getrocknet ($MgSO_4$) filtriert und eingeengt. Der Rückstand wird aus Ethanol umkristallisiert. Man erhält 39,06 g (81,5 mmol) = 85,6 % geschütztes Isothiobiuret vom

Fp: 169° C.

30 g (62mmol) dieses geschützten Isothiobiurets werden in 200 ml wasserfreiem Methanol gelöst, mit 168 mg (3,1 mmol) Natriummethanolat versetzt und unter Feuchtigkeitsausschluß bei Raumtemperatur gerührt. Es entsteht eine farblose Fällung von 1-β-Glucosyl-4-5-methylisothiobiuret. Diese wird abgesaugt und getrocknet. Man erhält 12,3 g (39,2 mmol) Produkt vom

FP: 184° C.

Ausbeute: 12,3g = 39,2 mmol = 63,2 % Fp: 184° C

Die Prüfung auf nematizide Wirkung (Testorganismus: Heterodesarostochiensis) ergab folgende Resultate:

Bei einer Produktkonzentration von 40 ppm: 100 % Wirkung

Bei einer Produktkonzentration von 20 ppm: 95 % Wirkung

**Patentansprüche**

1. Verfahren zur Herstellung von acylierten Zuckern mit glycosidisch gebundener Isothiocyanatgruppe aus acylierten Zuckern mit glycosidisch gebundenem Halogenatom, dadurch gekennzeichnet, daß man den acylierten Zucker mit glycosidisch gebundenem Halogenatom mit wenigstens der äquivalenten Menge eines Rhodanids der Formel

Me - NCS

in der

Me Alkali, Erdalkali oder Ammonium bedeutet, in einem inerten Lösungsmittel bei erhöhter Temperatur in Gegenwart eines Phasentransferkatalysators umsetzt.

2. Verfahren nach Anspruch 1, dadurch gekennzeichnet, daß man als Rhodanid Natrium-, Kalium- oder Ammonium-Rhodanid einsetzt.

3. Verfahren nach den Ansprüchen 1 und 2, dadurch gekennzeichnet, daß man 1 bis 2 Äqivalente des Rhodanids bezogen auf 1 Äqivalent des acylierten Zuckers mit glycosidisch gebundenem Halogenatom einsetzt.

4. Verfahren nach den Ansprüchen 1 bis 3, dadurch gekennzeichnet, daß ein Lösungsmittel eingesetzt wird, in dem sich das Rhodanid nicht löst.

5. Verfahren nach den Ansprüchen 1 bis 4, dadurch gekennzeichnet, daß ein Phasentransferkatalysator mit einer Komplexierungskonstante von 170 L/mol bis $10^8$ L/mol eingesetzt wird.

6. Verfahren nach den Ansprüchen 1 bis 5, dadurch gekennzeichnet, daß als Phasentransferkatalysator ein Polyether aus Ethylenoxidbausteinen eingesetzt wird.

7. Verfahren nach den Ansprüchen 1 bis 6, dadurch gekennzeichnet, daß die Wasserstoffatome der endständigen Hydroxygruppen des Polyethers durch Alkylreste ersetzt sind.

8. Verfahren nach den Ansprüchen 1 bis 6, dadurch gekennzeichnet, daß ein linearer Polyether mit einem durchschnittlichnen molekulargewicht von 200 bis 2000 eingesetzt wird.

9. Verfahren nach den Ansprüchen 1 bis 6, dadurch gekennzeichnet, daß ein makrocyclischer Polyether mit einer Ringgröße von 12 bis 24 Atomen eingesetzt wird.

**Claims**

1. Process for the preparation of acylated sugars with a glycosidically bonded isothiocyanate group from acylated sugars with a glycosidically bonded halogen atom, characterised in that the acylated sugar with a glycosidically bonded halogen atom is reacted with at least the equivalent amount of a thiocyanate of the formula

Me - NCS

in which

Me denotes an alkali metal, an alkaline earth metal or ammonium,

in an inert solvent at elevated temperature, in the presence of a phase transfer catalyst.

2. Process according to Claim 1, characterised in that sodium thiocyanate, potassium thiocyanate or ammonium thiocyanate is used as the thiocyanate.

3. Process according to Claims 1 and 2, characterised in that 1 to 2 equivalents of the thiocyanate are employed per equivalent of the acylated sugar with a glycosidically bonded halogen atom.

4. Process according to Claims 1 to 3, characterised in that a solvent in which the thiocyanate is insoluble is used.

5. Process according to Claims 1 to 4, characterised in that a phase transfer catalyst with a complexing constant of 170 L/mol to $10^8$ L/mol is used.

6. Process according to Claims 1 to 5, characterised in that a polyether of ethylene oxide units is used as the phase transfer catalyst.

7. Process according to Claims 1 to 6, characterised in that the hydrogen atoms of the terminal hydroxyl groups of the polyether are replaced by alkyl radicals.

8. Process according to Claims 1 to 6,

characterised in that a linear polyether with an average molecular weight of 200 to 2000 is used.

9. Process according to Claims 1 to 6, characterised in that a macrocyclic polyether with a ring size of 12 to 24 atoms is used.

**Revendications**

1. Procédé de production de sucres acylés porteurs d'un groupe isocyanate en liaison glucosidique à partir de sucres acylés porteurs d'un atome d'halogène en liaison glucosidique, caractérisé en ce qu'on fait réagir le sucre acylé porteur d'un atome d'halogène en liaison glucosidique avec au moins la quantité équivalente d'un sulfocyanure de formule

Me - NCS

dans laquelle

Me désigne un métal alcalin, un métal alcalinoterreux ou l'ion ammonium,

dans un solvant inerte, à température élevée, en présence d'un catalyseur de transfert de phase.

2. Procédé suivant la revendication 1, caractérisé en ce qu'on utilise comme sulfocyanure le sulfocyanure de sodium, de potassium ou d'ammonium.

3. Procédé suivant les revendications 1 et 2, caractérisé en ce qu'on utilise 1 à 2 équivalents de sulfocyanure pour 1 équivalent du sucre acylé porteur d'un atome d'halogène en liaison glucosidique.

4. Procédé suivant les revendications 1 à 3, caractérisé en ce qu'on utilise un solvant dans lequel le sulfocyanure ne se dissout pas.

5. Procédé suivant les revendications 1 à 4, caractérisé en ce qu'on utilise un catalyseur de transfert de phase ayant une constante de complexation de 170 L/mole à $10^8$ L/mole.

6. Procédé suivant les revendications 1 à 5, caractérisé en ce qu'on utilise comme catalyseur de transfert de phase un polyéther constitué de motifs d'oxyde d'éthylène.

7. Procédé suivant les revendications 1 à 6, caractérisé en ce que les atomes d'hydrogène des groupes hydroxy terminaux du polyéther sont remplacés par des restes alkyle.

8. Procédé suivant les revendications 1 à 6, caractérisé en ce qu'on utilise un polyéther linéaire ayant un poids moléculaire moyen de 200 à 2000.

9. Procédé suivant les revendications 1 à 6, caractérisé en ce qu'on utilise un polyéther macrocyclique dont le noyau comporte 12 à 24 atomes.